# EUROPEAN PATENT SPECIFICATION

(11) **EP 2 002 850 B1**
(45) Date of publication and mention of the grant of the patent: **05.04.2017**
(21) Application number: 08251790.5
(22) Date of filing: 22.05.2008
(51) Int. Cl.: A61L 24/04

(54) **Adhesive formulations**
Haftformulierungen
Formules adhésives

(30) Priority: 24.05.2007 US 931571 P
(43) Date of publication of application: 17.12.2008
(73) Proprietor: Covidien LP, Mansfield, MA 02048 (US)
(72) Inventor: Kapiamba, Mbiya, Cromwell, CT 06416 (US); Hadba, Ahman R., Wallingford, CT 06492 (US)
(74) Representative: Soames, Candida Jane

(56) References cited:
- EP-B1- 2 049 167
- WO-A-01/00246
- WO-A-01/16210
- WO-A-2007/067623
- US-A1- 2003 032 734
- US-A1- 2005 142 162

## Description

### TECHNICAL FIELD

The present disclosure relates to methods of making adhesives and sealants formed from synthetic components for medical and surgical use with animal tissues in vivo.

### BACKGROUND OF RELATED ART

In recent years there has developed an increased interest in replacing or augmenting sutures with adhesive bonds. The reasons for this increased interest include: (1) the potential speed with which repair might be accomplished; (2) the ability of a bonding substance to effect complete closure, thus preventing seepage of fluids; and (3) the possibility of forming a bond without excessive deformation of tissue or additional injury to tissue.

Studies in this area, however, have revealed that in order for surgical adhesives to be accepted by surgeons, they should possess various properties. For example, they should exhibit high initial tack and an ability to bond rapidly to living tissue; the strength of the bond should be sufficiently high to cause tissue failure before bond failure; the adhesive should form a bridge, typically a permeable flexible bridge; and the adhesive bridge and/or its metabolic products should not cause local histotoxic or carcinogenic effects.

Several materials useful as tissue adhesives or tissue sealants are currently available. One type of adhesive that is currently available is a cyanoacrylate adhesive. However, there is the possibility that a cyanoacrylate adhesive can degrade to generate undesirable by-products such as formaldehyde. Another disadvantage with cyanoacrylate adhesives is that they can have a high elastic modulus which can limit their usefulness.

Another type of tissue sealant that is currently available utilizes components derived from bovine and/or human sources. For example, fibrin sealants are available. However, as with any natural material, variability in the material is frequently observed and, because the sealant is derived from natural proteins, there may be viral transmission concerns.

It would be desirable to provide a biological adhesive or sealant that is fully synthetic and therefore highly consistent in its properties without the concern of viral transmission. Such a composition should be flexible and biocompatible and should be suitable for use as an adhesive or sealant.

US2005/0142162A describes various soft tissue implants containing therapeutic compounds. In one embodiment the therapeutic agent is released from a crosslinked matrix formed by a self-reactive compound. The self-reactive compound comprises a core substituted with a minimum of three reactive groups, wherein at least one of the reactive groups can undergo a bond-forming reaction with at least one of the other reactive groups.

WO2007/067623A, which forms part of the state of the art by virtue of Art. 54(3) EPC, describes polymeric components for reaction with polyamines to form medical adhesives and sealants. The polymeric components of have a triol core having polyalkylene arms functionalised with isocyanate groups.

US2003/0032734A describes two-component composition that react to form medical adhesives. The compositions may contain a component which is a bioabsorbable polyalkylene oxide having amine substituents, or a bioabsorbable polyalkylene oxide having isocyanate substituents.

WO01/16210A describes compositions that form interpenetrating polymer networks for use as high-strength tissue sealants.

WO01/00246A describes various polyalkylene oxides functionalised with activated carboxylate groups or isocyanate groups for reaction with chitosan to form crosslinked polymers.

EP2049167A, which forms part of the state of the art by virtue of Art. 54(3) EPC, describes a medical adhesive or sealant composition that is cured by moisture. The composition comprises a precursor compound that is a branched polyethylene oxide having terminal imine groups that reacts in the presence of moisture with a polyalkylene oxide having isocyanate or succinimide ester groups.

### SUMMARY

In a first aspect, the present invention provides a method as defined in claim 1 of making a biocompatible component for reaction with a polyfunctional amine cross linker for the preparation of a medical adhesive composition.

In a second aspect, the present invention provides a biocompatible component obtainable by the method of the invention.

In a third aspect, the present invention provides a method of making a medical adhesive composition comprising combining the biocompatible component of the invention with a polyfunctional amine cross linker.

Preferred features of the invention are recited in the dependent claims.

The compositions obtainable by the methods of the present disclosure can be applied by a variety of methods, including spraying the compositions onto a surgical site. In embodiments, the present disclosure includes methods for closing wounds by applying a composition of the present disclosure to a wound and allowing the composition to set, thereby closing said wound. Such wounds may include, in embodiments, incisions. Compositions of the present disclosure may also be utilized to seal leaks in animal. In embodiments, compositions of the present disclosure may also be utilized to adhere a medical device, such as an implant, to a surface of animal tissue.

### DETAILED DESCRIPTION

The present disclosure relates to methods of making biocompatible compositions for use as tissue adhesives or sealants, which are biocompatible, non-immunogenic and biodegradable. The biocompatible compositions can be employed to approximate tissue edges, adhere medical devices (e.g. implants) to tissue, seal air/fluid leaks in tissues, and for tissue augmentation such as sealing or filling voids or defects in tissue. Thus, as used herein, an "adhesive" is understood to include a composition which adheres one thing to another, such as tissue edges to each other or a device, such as an implant, to tissue, and a "sealant" is understood to include a composition which is applied to tissue and utilized to seal air/fluid leaks in tissue or seal or fill small voids or defects in tissue. However, an adhesive composition herein may be used as a sealant, and a sealant composition may be used as an adhesive.

The biocompatible compositions can be applied to living tissue and/or flesh of animals, including humans. While certain distinctions may be drawn between the usage of the terms "flesh" and "tissue" within the scientific community, the terms are used interchangeably herein as referring to a general substrate upon which those skilled in the art would understand the present composition to be utilized within the medical field for the treatment of patients. As used herein, "tissue" may include, but is not limited to, skin, bone, neuron, axon, cartilage, blood vessel, cornea, muscle, fascia, brain, prostate, breast, endometrium, lung, pancreas, small intestine, blood, liver, testes, ovaries, cervix, colon, stomach, esophagus, spleen, lymph node, bone marrow, kidney, peripheral blood, embryonic tissue, and/or ascite tissue.

The present invention provides a method of making a biocompatible component for reaction with a polyfunctional amine cross linker for the preparation of a medical adhesive composition, the method comprising the steps of: providing a multifunctional core compound, wherein the multifunctional core compound has from 3 to 12 functional groups and is either: (a) a polyol having molecular weight of from 130 to 20,000 g/mol and selected from the group consisting of polyether polyols, polyester polyols, branched chain ethoxylated alcohols, alkoxylated alcohols, polyvinyl alcohols, polyhydric alcohols, carboxylic acid esters of polyhydric alcohols, polyglycols, polylactone polyols, and combinations thereof, or (b) a multifunctional amine having molecular weight of from 132 to 10,000 g/mol; reacting the hydroxyl or amino groups of the multifunctional core compound with an aromatic, aliphatic or alicyclic diisocyanate thereby forming a multifunctional core possessing isocyanate-functionalized arms; followed by reacting the isocyanate groups of the isocyanate-functionalized arms with a polyalkylene oxide to attach polyalkyene oxide groups thereto, wherein the polyalkylene oxide is selected from the group consisting of polyethylene glycols, polypropylene glycols, polyethylene oxides, polypropylene oxides, polyethylene glycols with lactide linkages, polypropylene glycol-co-polyethylene oxide copolymers, polyethylene oxide/polypropylene oxide copolymers, and combinations thereof; followed by converting free hydroxyl groups at the ends of the polyalkylene oxide groups to terminal carboxylic groups by reacting them with an anhydride; and converting the terminal carboxylic groups to activated ester groups by reacting with N-hydroxysuccinimide or N-hydroxysulfosuccinimide, whereby the resulting biocompatible component has from 3 to 12 arms having said polyalkylene oxide groups functionalized with activated ester groups.

Multifunctional polyols which may be utilized to form a multifunctional core in accordance with the present disclosure are selected from polyether polyols; polyester polyols; block copolymers including branched chain ethoxylated alcohols; alkoxylated alcohols such as NEODOL^{®} which is sold commercially by Shell Chemical Company; polyvinyl alcohols; polyhydric alcohols; carboxylic acid esters of polyhydric alcohols; polyglycols; polylactone polyols; and combinations thereof.

In some embodiments, suitable polyols for use as the multifunctional polyol include glycerol, pentaerythritol, sorbitol, mannitol, trimethylol propane, diethylene glycol, pentaerythritol ethoxylate, pentaerythritol propoxylate, dipentaerythritiol, hexane-1,2,6-triol, or polycaprolactone triol.

Where the multifunctional core is a multifunctional polyol, the polyol has a molecular weight of from 130 g/mol to 20,000 g/mol, in embodiments from about 134 g/mol to about 1000 g/mol.

Examples of multifunctional amines which may be utilized to form a multifunctional core in accordance with the present disclosure include, but are not limited to, poly(allyl amine), poly(L-lysine), polyalkylene oxides having three or more amine functional groups, polyethylene oxide/polypropylene oxide copolymers possessing three or more amine functional groups, trilysine, diethylene triamine, di(heptamethylene) triamine, di(trimethylene) triamine, bis(hexamethylene) triamine, triethylene tetramine, tripropylene tetramine, tetraethylene pentamine, hexamethylene heptamine, pentaethylene hexamine, dimethyl octylamine, dimethyl decylamine, rh-collagen, rh-gelatin, chitosan, and combinations thereof.

Where the multifunctional core is a multifunctional amine, the amine has a molecular weight of from 132 g/mol to 10,000 g/mol.

The polyalkylene oxides which are combined with a multifunctional core are selected from polyethylene glycols ("PEG"), polypropylene glycols ("PPG"), polyethylene oxides ("PEO"), polypropylene oxides ("PPO"), polyethylene glycols with lactide linkages, polypropylene glycol-co-polyethylene oxide block or random copolymers, polyethylene oxide/polypropylene oxide copolymers, sometimes referred to herein as PEO/PPO copolymers or poloxamers, including triblock PEO/PPO copolymers commercially available as PLURONICS^{®} from BASF Corporation (Mt. Olive, NJ), and combinations thereof.

As noted above, the multifunctional core is combined with an aromatic, aliphatic or alicyclic diisocyanate. Examples include, but are not limited to, aromatic diisocyanates such as 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenyl isocyanate), and/or 2,4,6-trimethyl-1,3-phenylene diisocyanate; aliphatic diisocyanates such as tetramethylxylylene diisocyanate, tetramethylene diisocyanate, hexamethylene diisocyanate, lysine diisocyanate, 2-methylpentane-1,5-diisocyanate, 3-methylpentane-1,5-diisocyanate, hexane-1,6-diisocyanate, and/or 2,2,4-trimethylhexamethylene diisocyanate; and alicyclic diisocyanates such as isophorone diisocyanate, cyclohexane diisocyanate, hydrogenated xylylene diisocyanate, hydrogenated diphenylmethane diisocyanate, and/or hydrogenated trimethylxylylene diisocyanate. In embodiments, combinations of the foregoing isocyanates may be utilized.

In some embodiments, isocyanates which may be combined with the multifunctional cores include, but are not limited to, toluene diisocyanate (TDI), 4,4'-diphenylmethane diisocyanate (MDI), isophorone diisocyanate (IPDI), hexamethylene diisocyanate (HMDI), m-tetramethylxylylene diisocyanate (m-TMXDI), p-tetramethylxylylene diisocyanate (p-TMXDI), and combinations thereof.

The multifunctional core is combined with multiple groups forming arms thereon, using both isocyanates and polyalkylene oxides noted above. Methods for combining such components are within the purview of those skilled in the art.

The multifunctional core is first combined with a diisocyanate, thereby forming a multifunctional core possessing isocyanate arms. The free isocyanate group of the diisocyanate is then reacted with a polyalkylene oxide as described above, thereby forming arms having a diisocyanate adjacent the multifunctional core, followed by a polyalkylene oxide.

The free hydroxyl groups of the polyalkylene oxide groups furthest from the multifunctional core are then further functionalized. Specifically, the free hydroxyl groups are converted to carboxylic groups by reacting them with anhydrides such as succinic anhydride in the presence of tertiary amines such as pyridine or triethylamine or dimethylaminopyridine ("DMAP"). Other anhydrides which may be utilized include, but are not limited to, glutaric anhydride, phthalic anhydride, maleic anhydride, and combinations thereof. The resultant terminal carboxyl groups are then converted to an activated ester by reacting with N-hydroxysuccinimide (NHS) and/or N-hydroxysulfosuccinimide (Sulfo-NHS), optionally in the presence of dicyclohexylcarbodiimide (DCC) and/or N-(3-dimethylaminopropyl) carbodiimide (EDC), to produce N-hydroxysuccinimide ester groups, which are electrophilic, at the ends of the arms of the biocompatible component of the present disclosure.

In embodiments, the multifunctional core, the arms, or both, may include degradable linkages so as to render the components of the present disclosure degradable, as well as any composition including these components. Suitable degradable linkages which can be optionally incorporated in the biocompatible component and/or compositions of the present disclosure include, but are not limited to, hydrolytically labile -hydroxy acids such as lactic acid, glycolic acid, and hydroxy-butyric acid, glycolide, lactide, lactones including - caprolactone, carbonates such as trimethylene carbonate, ester ethers such as dioxanones including 1,4-dioxane-2-one and 1,3-dioxane-2-one, diacids including succinnic acid, adipic acid, sebacic acid, malonic acid, glutaric acid, azelaic acid, phosphoesters such as ethyl dichlorophosphate, anhydrides including sebacic acid anhydride and azelaic acid anhydride, combinations thereof. Those skilled in the art will readily envision reaction schemes for incorporating these degradable linkages into the biocompatible component of the present disclosure.

The electrophilic groups at the ends of the arms of the biocompatible component of the present disclosure may then be reacted with an amine cross linker to produce an adhesive or sealant composition in accordance with the present disclosure. As would be readily apparent to one skilled in the art, the desired properties of the compositions of the present disclosure can be adjusted by the selection of the specific components utilized to prepare the resulting adhesive or sealant compositions.

Suitable amine crosslinkers which may be reacted with the biocompatible component of the present disclosure include those multifunctional amines described above which may be used as the multifunctional core. Amine cross linkers which may be utilized include, for example, primary amines, secondary amines, diamines, aromatic amines, polyamines, polyamidoamines, and combinations thereof. Multifunctional amines may also include primary aliphatic amines, primary aromatic amines, secondary aliphatic or alicyclic amines, and/or secondary aromatic amines. The amine group may be linked to the multifunctional cores by other groups such as ester, amide, ether, amine, combinations thereof, and the like. Suitable amines which may be utilized as the amine cross linker include poly(allyl amine), poly(L-lysine), polyalkylene oxides having two or more primary or secondary amine functional groups, spermidine, spermine, 1,4-bis(3-aminopropyl)piperazine, and diaminobicyclooctane.

Other examples of suitable amines which may be used as the at least one amine cross linker include, but are not limited to, triethylamine, diisopropylethylamine, ethylene diamine, 1,4-butane diamine, hexamethylene diamine, isomers of hexamethylene diamine, diethylene triamine, triethylene tetramine, lysine and lysine containing polypeptides, arginine and arginine containing polypeptides, tetraethylene pentamine, bishexamethylene triamine, N,N'-Bis(3-aminopropyl)-1,2-ethane diamine, N-(3-Aminopropyl)-1,3-propane diamine, N-(2-aminoethyl)-1,3 propane diamine, cyclohexane diamine, isomers of cyclohexane diamine, 4,4'-methylene biscyclohexane amine, 4'4'-methylene bis(2-methylcyclohexane amine), isophorone diamine, phenalkylene polyamines, and combinations thereof.

Aromatic amines may also be used as the amine cross linker. Suitable aromatic amines include, for example, di-(4-aminophenyl)sulfone, di-(4-aminophenyl)ether, 2,2-bis(4-aminophenyl)propane, 4,4'-diamino diphenylmethane, 3,3'-dimethyl-4,4'-diaminodiphenyl methane, m-phenylene diamine, p-phenylene diamine, m-xylylene diamine, toluene diamine, 4,4'-methylene dianiline, benzidine, 4,4'-thiodianiline, 4-methoxy-1,3-phenyldiamine, 2,6-diaminopyridine, dianisidine, and combinations thereof.

Polyether diamines may also be utilized as the amine cross linker. Suitable polyether diamines include, but are not limited to, 4,9-dioxadodecane-1,12-diamine, 4,7,10-trioxatridecane-1,12-diamine, bis(3-amino propyl)polytetrahydrofurans, Bis(3-aminopropyl)amine, 1,2-Bis(3-aminopropylamino)ethane, and commercially available polyoxyalkylene amines from Texaco Chemical Co. under the JEFFAMINE^{®} brand as D230, D400, D2000, T403, and T-3000. Combinations of the foregoing polyether diamines may be utilized in embodiments.

In some embodiments, the amine cross linker can be an amino functional polymer such as those sold under the JEFFAMINE^{®} brand, a poly(allyl amine), poly(L-lysine), or other amino functional polymers such as a polyalkylene oxide, including PEG, PEO and PPO having two or more amine functional groups.

Other suitable amine cross linkers include chitosan, recombinant proteins such as rh-collagen, rh-gelatin and rh-albumin, recombinant glycosaminoglycans such as rh-hyaluronic acid, combinations thereof, and the like.

In embodiments, combinations of the foregoing cross linkers may be utilized to form an adhesive composition and/or sealant composition of the present disclosure.

An adhesive composition and/or sealant composition of the present disclosure may thus possess the biocompatible component of the present disclosure in an amount of from 10 to 100 percent by weight of the composition, in embodiments from 50 to 90 percent by weight of the composition, with the cross linker component of the adhesive composition and/or sealant composition present in an amount of from 0 to 90 percent by weight of the composition, in embodiments from 10 to 50 percent by weight of the composition.

In some embodiments, the weight ratio of the biocompatible component of the present disclosure to the cross linker in a composition of the present disclosure may be from 5000:1 to 2.5:1, in embodiments from 1000:1 to 10:1.

The resulting composition of the present disclosure can be used in a medical/surgical capacity in place of, or in combination with, sutures, staples, clamps, combinations thereof, and the like.

Optional components may be added to the composition of the present disclosure to adjust its viscosity according to a specific application of use, e.g., as an adhesive or a sealant. Such optional components can include, for example, diethylene glycol dimethyl ether ("DIGLYME"), dimethylformamide ("DMF"), dimethyl succinate, dimethyl glutarate, dimethyl adipate, combinations thereof, and the like. Thickening agents which can be used to adjust the viscosity of the compositions of the present disclosure include polycyanoacrylates, polylactic acid, polyglycolic acid, lactic-glycolic acid copolymers, poly-3-hydroxybutyric acid, polyorthoesters, polyanhydrides, pectin, combinations thereof, and the like.

Where utilized, such additives can be included so that they are present in an amount of from 1 to 30 percent by weight of the composition, in embodiments from 2 to 15 percent by weight of the composition.

Optionally, stabilizers can also be added to increase the storage stability of the compositions of the present disclosure. Suitable stabilizers can include those which prevent premature polymerization such as quinones, hydroquinone, hindered phenols, hydroquinone monomethyl ether, catechol, pyrogallol, benzoquinone, 2-hydroxybenzoquinone, p-methoxy phenol, t-butyl catechol, butylated hydroxy anisole, butylated hydroxy toluene, t-butyl hydroquinone, and combinations thereof. Suitable stabilizers can also include anhydrides, silyl esters, sultones (e.g., α-chloro-α-hydroxy-o-toluenesulfonic acid-γ-sultone), sulfur dioxide, sulfuric acid, sulfonic acid, sulfurous acid, lactone, boron trifluoride, organic acids, alkyl sulfate, alkyl sulfite, 3-sulfolene, alkylsulfone, alkyl sulfoxide, mercaptan, alkyl sulfide, and combinations thereof. In some embodiments, an anhydride such as maleic anhydride, sebacic acid anhydride, and/or azelaic acid anhydride, can be used as a stabilizer. In other embodiments antioxidants such as Vitamin E, Vitamin K1, cinnamic acid, and/or flavanone can be used as stabilizers.

Where utilized, such stabilizers can be included so that they are present in an amount from 0.01 to 10 percent by weight of the composition, in embodiments from 0.1 to 2 percent by weight of the composition.

In some embodiments, solid supported catalysts may be used during synthesis to improve stability of the resulting composition of the present disclosure. The presence of such catalysts may increase reactivity during use. Suitable catalysts are within the purview of those skilled in the art and can include stannous octoate, triethylamine, diethylaminoethanol, dimethylaminopyridine (DMAP), combinations thereof, and the like. The amount of catalyst employed can be from 0.5 grams to 50 grams per kilogram of the other components of the composition.

The compositions obtainable by the methods of the present disclosure can be used for a number of different human and animal medical applications including, but not limited to, wound closure (including surgical incisions and other wounds), adhesives for medical devices (including implants), void fillers, and embolic agents. Adhesive compositions and/or sealant compositions may be used to bind tissue together either as a replacement of, or as a supplement to, sutures, staples, clamps, tapes, bandages, and the like. Use of the disclosed compositions can eliminate or substantially reduce the number of sutures normally required during current practices, and eliminate the subsequent need for removal of staples and certain types of sutures. The compositions of the present disclosure thus can be particularly useful for use with delicate tissues where sutures, clamps or other conventional tissue closure mechanisms may cause further tissue damage.

Application of the compositions of the present disclosure, with or without other additives, can be done by any conventional means. These include dripping, brushing, or other direct manipulation of the composition on the tissue surface, by syringe, such as with a mixer nozzle, or spraying of the composition onto the surface. In open surgery, application by hand, forceps, or the like is contemplated. In endoscopic surgery, the composition can be delivered through the cannula of a trocar, and spread at the site by any device within the purview of those skilled in the art.

In embodiments, the biocompatible component of the present disclosure, optionally in combination with the cross linker, may be dissolved in a solvent to form a solution for application. Suitable solvents include those that are water miscible and biologically acceptable for medical/surgical use. In some embodiments, the solvents can include DIGLYME (diethylene glycol dimethyl ether), N,N-dimethylformamide ("DMF"), dimethyl sulfoxide, and combinations thereof.

In embodiments, the biocompatible component may be in a first solution, with the at least one cross linker dissolved in an aqueous media which optionally contains at least one biodegradable thickener. Suitable biologically acceptable thickeners include disaccharides, polysaccharides, alginates, hyaluronic acid, pectins, dextrans, cellulosics such as carboxymethyl cellulose, methyl cellulose, combinations thereof, and the like.

The biocompatible component may be present in the first solution in an amount from 10% to 100% by weight of the first solution, in embodiments from 50% to 90% by weight of the first solution. The amount of cross linker in the aqueous media, sometimes referred to herein as a second solution, may be from 0.01 % to 10% by weight of the second solution, in embodiments from 0.05% to 5% by weight of the second solution. Where present, a biodegradable thickener may be present in an amount from about 0% to 10% by weight of the second solution.

The first component solution and the second cross linker solution may then be combined upon application to form a sealant or adhesive composition of the present disclosure. For example, the composition of the present disclosure can be dispensed from a conventional adhesive dispenser, which may provide mixing of the first and second components prior to the dispenser. Such dispensers are disclosed, for example, in U.S. Patent Nos. 4,978,336 , 4,361,055 , 4,979,942 , 4,359,049 , 4,874,368 , 5,368,563 , and 6,527,749.

In some embodiments, a dual-compartment applicator may be utilized and mixing of the first component solution and second component solution may occur to form an adhesive upon dispensing by an aerosol or by means of a mixing head attached to the applicator or syringe. Other additives can be introduced into the first component solution, the second component solution, or both.

For example, the adhesive composition may be sprayed onto mammalian tissue, which lowers the risk of additional mechanical stress on the tissue. The spray application can be by any means within the purview of those skilled in the art such that the composition can be applied as a fine mist or aerosol. For example, the composition can be placed in a spray bottle and delivered with a hand pump. Alternatively, the composition can be placed in a container with a non-chlorofluorohydrocarbon propellant (e.g., air, nitrogen, carbon dioxide, and/or hydrocarbons) and delivered using a pressurized spray can. In either case, the composition is passed through a fine orifice to form a mist and delivered to the surgical location.

In other embodiments, especially where the composition of the present disclosure is to be utilized as a void filler or to fill a defect in an animal's body, it may be advantageous to more precisely control the conditions and extent of cross-linking; in such a case, it may be desirable to partially cross-link the composition prior to its use to fill a void in animal tissue. The composition of the present disclosure may then be applied to the void or defect and allowed to set, thereby filling the void or defect.

To effectuate the joining of two tissue edges, the two edges may be approximated, and the biocompatible component may be applied in combination with the cross linker. In other embodiments, the biocompatible component may be applied to one tissue edge, the cross linker may be applied to a second tissue edge, and the two edges then brought into contact with each other. The components crosslink rapidly, generally taking less than one minute. The composition of the present disclosure can thus be used as an adhesive to close a wound, including a surgical incision. In such a case, the composition of the present disclosure can be applied to the wound and allowed to set, thereby closing the wound.

The adhesive composition of the present disclosure can be used to adhere a medical device to tissue, rather than secure two edges of tissue. In some aspects, the medical device includes an implant. Other medical devices include, but are not limited to, pacemakers, stents, shunts and the like. In some embodiments, depending on the composition of the medical device, a coating may be required on the medical device. In some aspects such a coating can include the biocompatible component of the present disclosure in combination with the cross linker. Generally, for adhering a device to the surface of animal tissue, the composition of the present disclosure can be applied to the device, the tissue surface, or both. In other embodiments, the biocompatible component of the present disclosure can be applied to either the device or the tissue surface, with the crosslinker applied to the other (i.e., where the biocompatible component has hot been applied). The device and tissue surface are then brought into contact with each other and the composition is allowed to set, thereby adhering the device and tissue surface to each other.

The composition of the present disclosure can also be used to prevent post surgical adhesions. In such an application, the composition may be applied and cured as a layer on surfaces of internal tissues in order to prevent the formation of adhesions at a surgical site during the healing process. In addition to the formation of adhesion barriers, in embodiments the adhesive may be utilized to form implants such as gaskets, buttresses or pledgets for implantation.

In another embodiment, the composition can be used to attach skin grafts and position tissue flaps during reconstructive surgery. In still another embodiment, the composition can be used to close tissue flaps in periodontal surgery.

Applications for the compositions of the present disclosure also include sealing tissues to prevent or control blood or other fluid leaks at suture or staple lines. In embodiments, the composition can be used to seal or adhere delicate tissue together in place of conventional tools that may cause mechanical stress. The composition can also be used to seal air and/or fluid leaks in tissue. Additionally, the composition can be applied to tissue as a barrier to prevent adhesions, provide a protective layer for delicate damaged tissue and/or provide a drug delivery layer to a surgical site.

When used as a sealant, the composition of the present disclosure can be used in surgery to prevent or inhibit bleeding or fluid leakage both during and after a surgical procedure. It can also be applied to prevent air leaks associated with pulmonary surgery. The sealant may be applied directly to the desired area in at least an amount necessary to seal off any defect in the tissue and seal off any fluid or air movement.

A variety of optional ingredients including medicinal agents may also be added to the compositions of the present disclosure. These agents may be added to adhesive compositions of the present disclosure, sealant compositions of the present disclosure, or both. A phospholipid surfactant that provides antibacterial stabilizing properties and helps disperse other materials in the compositions may be added to the compositions of the present disclosure. Additional medicinal agents include antimicrobial agents, colorants, preservatives, or medicinal agents such as, for example, protein and peptide preparations, antipyretic, antiphlogistic and analgesic agents, anti-inflammatory agents, vasodilators, antihypertensive and antiarrhythmic agents, hypotensive agents, antitussive agents, antineoplastics, local anesthetics, hormone preparations, antiasthmatic and antiallergic agents, antihistaminics, anticoagulants, antispasmodics, cerebral circulation and metabolism improvers, antidepressant and antianxiety agents, vitamin D preparations, hypoglycemic agents, antiulcer agents, hypnotics, antibiotics, antifungal agents, sedative agents, bronchodilator agents, antiviral agents, dysuric agents, and combinations thereof.

Imaging agents such as iodine, barium sulfate, or fluorine, can also be combined with the compositions of the present disclosure to allow visualization of the surgical area through the use of imaging equipment, including X-ray, MRI, and/or CAT scan.

Additionally, an enzyme may be added to the compositions of the present disclosure to increase their rate of degradation. Suitable enzymes include, for example, peptide hydrolases such as elastase, cathepsin G, cathepsin E, cathepsin B, cathepsin H, cathepsin L, trypsin, pepsin, chymotrypsin, and γ-glutamyltransferase ( γ-GTP); sugar chain hydrolases such as phosphorylase, neuraminidase, dextranase, amylase, lysozyme, oligosaccharase, and the like; oligonucleotide hydrolases such as alkaline phosphatase, endoribonuclease, and endodeoxyribonuclease. In some embodiments, where an enzyme is added, the enzyme may be included in a liposome or microsphere to control the rate of its release, thereby controlling the rate of degradation of the composition of the present disclosure. Methods for incorporating enzymes into liposomes and/or microspheres are within the purview of those skilled in the art.

The present compositions have a number of advantageous properties. The resulting compositions of the present disclosure are safe and biocompatible, possess enhanced adherence to tissue, are biodegradable, have hemostatic potential, have low cost, and are easy to prepare and use. The composition has a rapid curing time. Application of the composition, with or without other additives, can be done by any conventional means. By varying the selection of the components, the strength and elasticity of the adhesive and/or sealant composition can be controlled, as can the gelation time.

The compositions rapidly form a compliant gel matrix, which insures stationary positioning of tissue edges or implanted medical devices in the desired location where the composition is utilized as an adhesive, and a tightly adherent yet flexible seal where the composition is used as a sealant. In either case, the rapidity of gelation lowers the overall required surgical/application time. Where delicate or spongy tissues are involved and/or air or fluid leaks must be sealed, spray application of a composition may be utilized to avoid stress to the tissue and insure a uniform coating over the area.

The compositions retain the positional integrity of the tissue to which the composition is applied and/or location of a medical device. The compositions form strong cohesive bonds. They exhibit excellent mechanical performance and strength, while retaining the necessary pliability to adhere living tissue. This strength and pliability allows a degree of movement of tissue without shifting the surgical tissue edge. Additionally, the compositions are biodegradable, allowing the degradation components to pass safely through the subject's body.

It will be understood that various modifications may be made to the embodiments disclosed herein. For example, the compositions in accordance with this disclosure can be blended with other biocompatible, bioabsorbable or non-bioabsorbable materials. As another example, optional ingredients such as dyes, fillers, medicaments or antimicrobial compounds can be added to the composition. Those skilled in the art will envision other modifications within the scope of the claims appended hereto.

## Claims

1. A method of making a biocompatible component for reaction with a polyfunctional amine cross linker for the preparation of a medical adhesive composition, the method comprising the steps of:
providing a multifunctional core compound, wherein the multifunctional core compound has from 3 to 12 functional groups and is either: (a) a polyol having molecular weight of from 130 to 20,000 g/mol and selected from the group consisting of polyether polyols, polyester polyols, branched chain ethoxylated alcohols, alkoxylated alcohols, polyvinyl alcohols, polyhydric alcohols, carboxylic acid esters of polyhydric alcohols, polyglycols, polylactone polyols, and combinations thereof, or (b) a multifunctional amine having molecular weight of from 132 to 10,000 g/mol;
reacting the hydroxyl or amino groups of the multifunctional core compound with an aromatic, aliphatic or alicyclic diisocyanate thereby forming a multifunctional core possessing isocyanate-functionalized arms; followed by
reacting the isocyanate groups of the isocyanate-functionalized arms with a polyalkylene oxide to attach polyalkyene oxide groups thereto, wherein the polyalkylene oxide is selected from the group consisting of polyethylene glycols, polypropylene glycols, polyethylene oxides, polypropylene oxides, polyethylene glycols with lactide linkages, polypropylene glycol-co-polyethylene oxide copolymers, polyethylene oxide/polypropylene oxide copolymers, and combinations thereof; followed by
converting free hydroxyl groups at the ends of the polyalkylene oxide groups to terminal carboxylic groups by reacting them with an anhydride; and
converting the terminal carboxylic groups to activated ester groups by reacting with N-hydroxysuccinimide or N-hydroxysulfosuccinimide,
whereby the resulting biocompatible component has from 3 to 12 arms having said polyalkylene oxide groups functionalized with activated ester groups.

2. The method of claim 1, wherein the multifunctional polyol is selected from the group consisting of hexane-1,2,6-triol, polycaprolactone triol, glycerol, pentaerythritol, sorbitol, mannitol, trimethylol propane, diethylene glycol, pentaerythritol ethoxylate, pentaerythritol propoxylate, dipentaerythritol, and combinations thereof.

3. The method of claim 1 or 2 , wherein the multifunctional amine core compound is selected from the group consisting of poly(allyl amine), poly(L-lysine), polyalkylene oxides having three or more amine groups, polyethylene oxide/polypropylene oxide copolymers possessing three or more amine groups, trilysine, diethylene triamine, di(heptamethylene) triamine, di(trimethylene) triamine, bis(hexamethylene) triamine, triethylene tetramine, tripropylene tetramine, tetraethylene pentamine, hexamethylene heptamine, pentaethylene hexamine, dimethyl octylamine, dimethyl decylamine, rh-collagen, rh-gelatin, chitosan, and combinations thereof.

4. The method of any one of the preceding claims, wherein the polyalkylene oxide is selected from the group consisting of polyethylene glycols, polypropylene glycols, polyethylene oxides, polypropylene oxides, polyethylene glycols with lactide linkages, polypropylene glycol-co-polyethylene oxide copolymers, polyethylene oxide/polypropylene oxide copolymers, and combinations thereof.

5. The method of any preceding claim, wherein the diisocyanate is selected from the group consisting of 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenyl isocyanate), 2,4,6-trimethyl-1,3-phenylene diisocyanate, tetramethylxylylene diisocyanate, tetramethylene diisocyanate, hexamethylene diisocyanate, lysine diisocyanate, 2-methylpentane-1,5-diisocyanate, 3-methylpentane-1,5-diisocyanate, hexane-1,6-diisocyanate, 2,2,4-trimethylhexamethylene diisocyanate, isophorone diisocyanate, cyclohexane diisocyanate, hydrogenated xylylene diisocyanate, hydrogenated diphenylmethane diisocyanate, hydrogenated trimethylxylylene diisocyanate, and combinations thereof.

6. A biocompatible component obtainable by a method according to any of claim 1 to 5.

7. A method of making a medical adhesive composition comprising combining the biocompatible component of claim 6 with a polyfunctional amine cross linker.

8. The method of claim 7, wherein the cross linker comprises an amine cross linker selected from the group consisting of poly(allyl amine), poly(L-lysine), polyalkylene oxides having two or more amine functional groups, spermidine, spermine, 1,4-bis(3-aminopropyl)piperazine, diaminobicyclooctane, triethylamine, diisopropylethylamine, ethylene diamine, 1,4-butane diamine, hexamethylene diamine, diethylene triamine, triethylene tetramine, lysine, lysine containing polypeptides, arginine, arginine containing polypeptides, tetraethylene pentamine, bishexamethylene triamine, N,N'-Bis(3-aminopropyl)-1,2-ethane diamine, N-(3-Aminopropyl)-1,3-propane diamine, N-(2-aminoethyl)-1,3 propane diamine, cyclohexane diamine, 4,4'-methylene biscyclohexane amine, 4'4'-methylene bis(2-methylcyclohexane amine), isophorone diamine, phenalkylene polyamines, di-(4-aminophenyl)sulfone, di-(4-aminophenyl)ether, 2,2-bis(4-aminophenyl)propane, 4,4'-diamino diphenylmethane, 3,3'-dimethyl-4,4'-diaminodiphenyl methane, m-phenylene diamine, p-phenylene diamine, m-xylylene diamine, toluene diamine, 4,4'-methylene dianiline, benzidine, 4,4'-thiodianiline, 4-methoxy-1,3-phenyldiamine, 2,6-diaminopyridine, dianisidine, 4,9-dioxadodecane-1,12-diamine, 4,7,10-trioxatridecane-1,12-diamine, bis(3-amino propyl)polytetrahydrofurans, Bis(3-aminopropyl)amine, 1,2-Bis(3-aminopropylamino)ethane, polyoxyalkylene amines, and combinations thereof.

9. The method of claim 7 or 8, wherein the biocompatible component is present in an amount from 50 to 90 percent by weight of the composition, and the cross linker is present in an amount from 10 to 50 percent by weight of the composition.

## Patentansprüche

1. Ein Verfahren zur Herstellung eines biokompatiblen Bestandteils für eine Reaktion mit einem polyfunktionalen Aminvernetzer für die Herstellung von einer medizinischen Klebstoffzusammensetzung, das Verfahren aufweisend die Schritte von:
Bereitstellen einer multifunktionalen Kernverbindung, wobei die multifunktionale Kernverbindung von 3 bis 12 funktionale Gruppen hat und entweder: (a) ein Polyol mit einem Molekulargewicht von 130 bis 20.000 g/Mol und ausgewählt aus der Gruppe bestehend aus Polyether-Polyole, Polyester-Polyole, verzweigtkettige ethoxylierte Alkohole, alkoxylierte Alkohole, Polyvinylalkohole, mehrwertige Alkohole, Karbonsäureester von mehrwertigen Alkoholen, Polyglykole, Polylacton-Polyole und Kombinationen davon oder (b) ein multifunktionales Amin mit einem Molekulargewicht von 132 bis 10.000 g/Mol ist; Reagieren der Hydroxyl- oder Aminogruppen von der multifunktionalen Kernverbindung mit einem aromatischen, aliphatischen oder alicyclischen Düsocyanat und dadurch Bilden eines multifunktionalen Kerns, der Isocyanat-funktionalisierte Arme hat; gefolgt von
Reagieren der Isocyanatgruppen von den Isocyanat-funktionalisierten Armen mit einem Polyalkylenoxid, um Polyalkenoxidgruppen daran zu befestigen, wobei das Polyalkylenoxid ausgewählt ist aus der Gruppe bestehend aus Polyethylenglykolen, Polypropylenglykolen, Polyethylenoxiden, Polypropylenoxiden, Polyethylenglykolen mit Lactid-Verbindungen, Polypropylen Glykol-co-polyethylenoxid-Copolymeren, Polyethylenoxid/Polypropylenoxid-Copolymeren und Kombinationen davon; gefolgt von
Konvertieren von freien Hydroxylgruppen an den Enden von den Polyalkylenoxidgruppen in terminale Carboxylgruppen durch Reagieren dieser mit einem Anhydrid; und
Konvertieren der terminalen Carboxylgruppen in aktivierte Estergruppen durch Reagieren mit N-Hydroxysuccinimid oder N-Hydroxysulfosuccinimid,
wobei der resultierende biokompatible Bestandteil von 3 bis 12 Arme hat, die die besagten Polyalkenoxidgruppen funktionalisiert mit aktivierten Estergruppen haben.

2. Das Verfahren nach Anspruch 1, wobei das multifunktionale Polyol ausgewählt ist aus der Gruppe bestehend aus Hexan-1,2,6-triol, Polycaprolactontriol, Glyzerin, Pentaerythrit, Sorbitol, Mannit, Trimethylolpropan, Diethylenglycol, Pentaerythritolethoxylat, Pentaerythritolpropoxylat, Dipentaerythritol und Kombinationen davon.

3. Das Verfahren nach Anspruch 1 oder 2, wobei die multifunktionale Aminkernverbindung ausgewählt ist aus der Gruppe bestehend aus Poly(allylamin), Poly(L-Lysin), Polyalkylenoxide mit drei oder mehr Amingruppen, Polyethylenoxid/Polypropylenoxid-Copolymere, die drei oder mehr Amingruppen haben, Trilysin, Diethylentriamin, Di(heptamethylen)triamin, Di(trimethylen)triamin, Bis(hexamethylen)triamin, Triethylentetramin, Tripropylentetramin, Tetraethylenpentamin, Hexamethylenheptamin, Pentaethylenhexamin, Dimethyloctylamin, Dimethyldecylamin, rh-Kollagen, rh-Gelatine, Chitosan und Kombinationen davon.

4. Das Verfahren nach irgendeinem der vorhergehenden Ansprüche, wobei das Polyalkylenoxid ausgewählt ist aus der Gruppe bestehend aus Polyethylenglykole, Polypropylenglykole, Polyethylenoxide, Polypropylenoxide, Polyethylenglykole mit Lactid-Verbindungen, Polypropylenglykol-co-polyethylenoxid-Copolymere, Polyethylenoxid/Polypropylenoxid-Copolymere und Kombinationen davon.

5. Das Verfahren nach einem vorangegangenen Anspruch, wobei das Diisocyanat ausgewählt ist aus der Gruppe bestehend aus 2,4-Toluoldiisocyanat, 2,6-Toluoldiisocyanat, 2,2'-Diphenylmethandiisocyanat, 2,4'-Diphenylmethandiisocyanat, 4,4'-Diphenylmethandiisocyanat, Diphenyldimethylmethandiisocyanat, Dibenzyldiisocyanat, Naphtylendiisocyanat, Phenylendiisocyanat, Xylylendiisocyanat, 4,4'-Oxybis(phenylisocyanat), 2,4,6-Trimethyl-1,3-phenylendiisocyanat, Tetramethylxylylendiisocyanat, Tetramethylendiisocyanat, Hexamethylendiisocyanat, Lysindiisocyanat, 2-Methylpentan-1,5-diisocyanat, 3-Methylpentan-1,5-diisocyanat, Hexan-1,6-diisocyanate, 2,2,4-Trimethylhexamethylendiisocyanat, Isophorondiisocyanat, Cyclohexandiisocyanat, hydriertes Xylylendiisocyanat, hydriertes Diphenylmethandiisocyanat, hydriertes Trimethylxylylendiisocyanat und Kombinationen davon.

6. Ein biokompatibler Bestandteil erhältlich durch ein Verfahren nach einem der Ansprüche 1 bis 5.

7. Ein Verfahren zur Herstellung einer medizinischen Klebstoffzusammensetzung, aufweisend Kombinieren des biokompatiblen Bestandteils nach Anspruch 6 mit einem polyfunktionalen Aminvernetzer.

8. Das Verfahren nach Anspruch 7, wobei der Vernetzer einen Aminvernetzer aufweist, der ausgewählt ist aus der Gruppe bestehend aus Poly(allylamin), Poly(L-Lysin), Polyalkylenoxide mit zwei oder mehr funktionalen Amingruppen, Spermidin, Spermin, 1,4-bis(3-aminopropyl)piperazin, Diaminobicyclooctan, Triethylamin, Diisopropylethylamin, Ethylendiamin, 1,4-Butandiamin, Hexamethylendiamin, Diethylentriamin, Triethylentetramin, Lysin, Polypeptide enthaltendes Lysin, Arginin, Polypeptide enthaltendes Arginin, Tetraethylenpentamin, Bishexamethylentriamin, N,N'-Bis(3-aminopropyl)-1,2-ethandiamin, N-(3-Aminopropyl)-1,3-Propandiamin, N-(2-aminoethyl)-1,3 Propandiamin, Cyclohexandiamin, 4,4'-Methylenbiscyclohexanamin, 4,4'-Methylen bis(2-methylcyclohexanamin), Isophorondiamin, Phenalkylenpolyamine, Di-(4-aminophenyl)sulfon, Di-(4-aminophenyl)ether, 2,2-bis(4-aminophenyl)propan, 4,4'-Diaminodiphenylmethan, 3,3'-Dimethyl-4,4'-diaminodiphenylmethan, m-Phenylendiamin, p-Phenylendiamin, m-Xylylendiamin, Toluoldiamin, 4,4'-Methylendianilin, Benzidin, 4,4'-Thiodianilin, 4-Methoxy-1,3-phenyldiamin, 2,6-Diaminopyridin, Dianisidin, 4,9-Dioxadodecan-1,12-diamin, 4,7,10-Trioxatridecan-1,12-diamin, bis(3-aminopropyl)polytetra-hydrofuran, Bis(3-aminopropyl)amin, 1,2-Bis(3-aminopropylamino)ethan, Polyoxyalkylenamine und Kombinationen davon.

9. Das Verfahren nach Anspruch 7 oder 8, wobei der biokompatible Bestandteil in einer Menge von 50 bis 90 Gewichtsprozent von der Zusammensetzung vorhanden ist und der Vernetzer in einer Menge von 10 bis 50 Gewichtsprozent von der Zusammensetzung vorhanden ist.

## Revendications

1. Procédé de fabrication d'un composant biocompatible pour une réaction avec un agent de réticulation d'amine polyfonctionnelle pour la préparation d'une composition adhésive médicale, le procédé comprenant les étapes consistant à :
fournir un composé d'âme multifonctionnel, dans lequel le composé d'âme multifonctionnelle a 3 à 12 groupements fonctionnels et est (a) un polyol ayant un poids moléculaire de 130 à 20 000 g/mole et choisi dans le groupe constitué de polyols de polyéthers, de polyols de polyesters, d'alcools éthoxylés à chaîne ramifiée, d'alcools alcoxylés, d'alcools polyvinyliques, d'alcools polyvalents, d'esters d'acides carboxyliques d'alcools polyvalents, de polyglycols, de polyols de polylactone et de leurs combinaisons, ou (b) une amine polyfonctionnelle ayant un poids moléculaire de 132 à 10 000 g/mole ;
faire réagir les groupements hydroxyle ou amino du composé d'âme multifonctionnelle avec un diisocyanate aromatique, aliphatique ou alicyclique, formant de la sorte une âme multifonctionnelle possédant des bras fonctionnalisés aux isocyanates ; puis
faire réagir les groupements isocyanate des bras fonctionnalisés aux isocyanates avec un poly(oxyde d'alkylène) pour y fixer des groupements de poly(oxyde d'alkylène), dans lequel le poly(oxyde d'alkylène) est choisi dans le groupe constitué de polyéthylèneglycols, de polypropylèneglycols, de poly(oxydes d'éthylène), de poly(oxydes de propylène) de polyéthylèneglycols avec des liaisons lactide, de copolymères de polypropylèneglycol et de poly(oxyde d'éthylène), de copolymères de poly(oxyde d'éthylène) et de poly(oxyde de propylène) et de leurs combinaisons ; le tout suivi de
la conversion de groupements hydroxyle libres aux extrémités des groupements de poly(oxyde d'alkylène) en groupements carboxyliques terminaux en les faisant réagir avec un anhydride ; et
la conversion des groupements carboxyliques terminaux en groupements ester activés par réaction avec du N-hydroxysuccinimide ou du N-hydroxysulfo-succinimide,
dans lequel le composant biocompatible obtenu a 3 à 12 bras ayant lesdits groupements de poly(oxyde d'alkylène) fonctionnalisés par des groupements ester activés.

2. Procédé selon la revendication 1, dans lequel le polyol multifonctionnel est choisi dans le groupe constitué de l'hexane-1,2,6-triol, du triol de polycaprolactone, du glycérol, du pentaérythritol, du sorbitol, du mannitol, du triméthylolpropane, du diéthylèneglycol, de l'éthoxylate de pentaérythritol, du propoxylate de pentaérythritol, du dipentaérythritol et de leurs combinaisons.

3. Procédé selon la revendication 1 ou 2, dans lequel le composé d'âme d'amine multifonctionnelle est choisi dans le groupe constitué de la poly(allylamine), de la poly(L-lysine), des poly(oxydes d'alkylène) ayant trois ou plus de groupements amine, de copolymères de poly(oxyde d'éthylène) et de poly(oxyde de propylène) possédant trois ou plus de groupements amine, de la trilysine, de la diéthylène-triamine, de la di(heptaméthylène)triamine, de la di(triméthylène)triamine, de la bis(hexaméthylène)triamine, de la triéthylènetétramine, de la tripropylène-tétramine, de la tétraéthylènepentamine, de l'hexaméthylène-heptamine, de la pentaéthylènehexamine, de la diméthyloctylamine, de la diméthyldécylamine, du rh-collagène, de la rh-gélatine, du chitosane et de leurs combinaisons.

4. Procédé selon l'une quelconque des revendications précédentes, dans lequel le poly(oxyde d'alkylène) est choisi dans le groupe constitué des polyéthylèneglycols, des polypropylèneglycols, des poly(oxydes d'éthylène), des poly(oxydes de propylène), des polyéthylèneglycols avec des liaisons lactide, des copolymères de polypropylèneglycol et de poly(oxyde d'éthylène), des copolymères de poly(oxyde d'éthylène) et de poly(oxyde de propylène) et de leurs combinaisons.

5. Procédé selon l'une quelconque des revendications précédentes, dans lequel ledit isocyanate est choisi dans le groupe constitué du diisocyanate de 2,4-toluène, du diisocyanate de 2,6-toluène, du diisocyanate de 2,2'-diphénylméthane, du ditisocyanate de 2,4'-diphénylméthane, du diisocyanate de 4,4'-diphénylméthane, du diisocyanate de diphényldiméthylméthane, du diisocyanate de dibenzyle, du diisocyanate de naphtylène, du diisocyanate de phénylène, du diisocyanate de xylylène, du 4,4'-oxybis(phénylisocyanate), du diisocyanate de 2,4,6-triméthyl-1,3-phénylène, du diisocyanate de tétraméthylxylylène, du diisocyanate de tétraméthylène, du diisocyanate d'hexaméthylène, du diisocyanate de lysine, du 2-méthylpentane-1,5-diisocyanate, du 3-méthylpentane-1,5-diisocyanate, de l'hexane-1,6-diisocyanate, du diisocyanate de 2,2,4-triméthylhexaméthylène, du diisocyanate d'isophorone, du diisocyanate de cyclohexane, du diisocyanate de xylylène hydrogéné, du diisocyanate de diphénylméthane hydrogéné, du diisocyanate de triméthylxylylène hydrogéné et de leurs combinaisons.

6. Composant biocompatible qui peut être obtenu par un procédé selon l'une quelconque des revendications 1 à 5.

7. Procédé de fabrication d'une composition adhésive médicale comprenant la combinaison du composé biocompatible de la revendication 6 avec un agent de réticulation d'amine polyfonctionnelle.

8. Procédé selon la revendication 7, dans lequel l'agent de réticulation comprend un agent de réticulation d'amine choisi dans le groupe constitué de la poly(allylamine), de la poly(L-lysine), des poly(oxydes d'alkylène) ayant deux ou plus de groupements fonctionnels amine, de la spermidine, de la spermine, de la 1,4-bis(3-aminopropyl)pipérazine, du diaminobicyclooctane, de la triéthylamine, de la diisopropyléthylamine, de l'éthylèneamine, de la 1,4-butanediamine, de l'hexaméthylènediamine, de la diéthylènetriamine, de la triéthylènetétramine, de la lysine, de la lysine contenant des polypeptides, de l'arginine, de l'arginine contenant des polypeptides, de la tétraéthylènepentamine, de la bishexaméthylènetriamine, de la N,N'-Bis(3-aminopropyl)-1,2-éthanediamine, de la N-(3-aminopropyl)-1,3-propanediamine, de la N-(2-aminoéthyl)-1,3-propane-diamine, de la cyclohexanediamine, de la 4,4'-méthylènebiscyclohexaneamine, de la 4'4'-méthylènebis(2-méthylcyclohexaneamine), de la diamine d'isophorone, des poly(amines de phénalkylène), de la di-(4-aminophényl)sulfone, du di-(4-aminophényl)éther, du 2,2-bis(4-aminophényl)propane, du 4,4'-diaminodiphényl-méthane, du 3,3'-diméthyl-4,4'-diaminodiphénylméthane, de la m-phénylène-diamine, de la p-phénylènediamine, de la m-xylylènediamine, de la toluène-diamine, de la 4,4'-méthylènedianiline, de la benzidine, de la 4,4'-thiodianiline, de la 4-méthoxy-1,3-phényldiamine, de la 2,6-diaminopyridine, de la dianisidine, de la 4,9-dioxadodécane-1,12-diamine, de la 4,7,10-trioxatridécane-1,12-diamine, des bis(3-aminopropyl)polytétrahydrofuranes, de la bis(3-aminopropyl)amine, du 1,2-Bis(3-aminopropylmino)éthane des polyoxyalkylèneamines et de leurs combinaisons.

9. Procédé selon la revendication 7 ou 8, dans lequel le composant biocompatible est présent en quantité de 50 à 90 pour cent en poids de la composition et l'agent de réticulation est présent en quantité de 10 à 50 pourcent en poids de la composition.
